(19) Europäisches Patentamt

European Patent Office

Office européen des brevets

(11) EP 0 975 310 B1

(12) FASCICULE DE BREVET EUROPEEN

(45) Date de publication et mention
de la délivrance du brevet:
**07.07.2004 Bulletin 2004/28**

(21) Numéro de dépôt: **98913824.3**

(22) Date de dépôt: **04.03.1998**

(51) Int Cl.⁷: $A61K\ 7/06$, $A61K\ 7/48$

(86) Numéro de dépôt international:
**PCT/FR1998/000423**

(87) Numéro de publication internationale:
**WO 1998/041184 (24.09.1998 Gazette 1998/38)**

(54) **COMPOSITION GELIFIEE VAPORISABLE**

VERSPRÜHBARE GELIERTE ZUSAMMENSETZUNG

VAPORIZABLE GELLED COMPOSITION

(84) Etats contractants désignés:
**DE ES FR GB IT**

(30) Priorité: **14.03.1997 FR 9703118**

(43) Date de publication de la demande:
**02.02.2000 Bulletin 2000/05**

(73) Titulaire: **L'OREAL**
**75008 Paris (FR)**

(72) Inventeur: **DUPUIS, Christine**
**F-75018 Paris (FR)**

(74) Mandataire: **Dodin, Catherine et al**
**L'OREAL - D.I.P.I.**
**25-29 Quai Aulagnier**
**92600 Asnières (FR)**

(56) Documents cités:
**EP-A- 0 551 748        EP-A- 0 551 749**
**WO-A-95/00105        WO-A-95/33436**
**WO-A-95/33437        US-A- 4 300 580**

**Description**

La présente invention concerne une nouvelle composition topique vaporisable constituée par un gel aqueux comprenant un matériau gélifiant hydrophile particulier. L'invention concerne également un dispositif constitué par un récipient contenant la composition cosmétique ci-dessus et un moyen de distribution de ladite composition, plus particulièrement un flacon pompe ou un dispositif aérosol.

Pour préparer des aérosols vaporisables dans toutes les positions on emploie actuellement des dispositifs particuliers coûteux, impliquant des solutions mécaniques complexes telles que des valves spécifiques ou des systèmes de poches dont la mise en oeuvre est peu pratique.

La Demanderesse a maintenant trouvé qu'en employant un gel présentant un profil rhéologique particulier, il était possible d'obtenir une composition gélifiée vaporisable par des équipements classiques, flacons pompes et aérosols, dans toutes les positions.

Les matériaux gélifiants usuels, comme les gommes de guar, les Carbopols® commercialisés par la Société GOODRICH, le Sepigel® 305 commercialisé par la Société SEPIC, ou les épaississants de type latex, comme les Acrysols® commercialisés par la Société ROHM & HAAS, ou le Viscoatex® 538 commercialisé par la société COATEX, conduisent à des gels ne présentant pas le profil rhéologique des gels selon l'invention et ne peuvent donc pas être vaporisés.

Il est en outre connu d'employer de l'alumine pour obtenir des gels dits « rhéofluidifiants » vaporisables (WO 94/16808). Toutefois, comme tous les gels de ce type décrits dans l'état de la technique, il est nécessaire d'agiter vigoureusement le flacon contenant la composition pour la fluidifier et rendre possible sa vaporisation.

les gels présentant le profil rhéologique particulier selon l'invention sont une solution aux problèmes exposés ci-dessus.

La présente invention concerne donc une composition constituée par un gel aqueux comprenant un matériau gélifiant hydrophile, le dit gel présentant le profil rhéologique suivant:

- une viscosité initiale $V_0$ comprise entre 3000 et 50000 Pa.s, la dite viscosité initiale $V_0$ étant stable jusqu'à une contrainte de cisaillement $C_1$,
- une viscosité $V_2$ après cisaillement à une contrainte $C_2$ pour laquelle le rapport $V_0/V_2$ est supérieur ou égal à 1000,
- la différence $C_2$-$C_1$ étant inférieure ou égale à 100 Pa.

Le gel a une viscosité initiale stable, c'est à dire constante sous de faibles contraintes de cisaillement de manière que la composition puisse être manipulée sans entraîner de modification importante de sa viscosité. Cette stabilité de la viscosité initiale est exprimée par une valeur de la viscosité du gel $V_1$ mesurée à la containte de cisaillement $C_1$ proche de $V_0$. Il est entendu que cette proximité doit être appréciée au regard de la chute de viscosité après cisaillement. De manière avantageuse, le rapport $V_0/V_1$ est inférieur ou égal à 2.

La contrainte de cisaillement $C_1$ est caractéristique de la force nécessaire pour obtenir une fluidification du gel, permettant en particulier sa vaporisation. De manière préférentielle, cette contrainte $C_1$ est supérieure ou égale à 50 Pa. L'homme du métier saura déterminer la valeur que ne devra pas dépasser cette contrainte en fonction de l'usage qu'il entend faire de la composition selon l'invention.

Pour une utilisation dans un dispositif permettant sa vaporisation, on peut illustrer ci-dessous les valeurs maximum de contrainte de cisaillement $C_1$ en fonction du dispositif de vaporisation.

Lorsque le moyen de distribution de la composition selon l'invention est un flacon pompe, la valeur maximum de contrainte de cisaillement $C_1$ est de préférence inférieure ou égale à 150 Pa.

Lorsque le moyen de distribution de la composition selon l'invention est un dispositif aérosol, la valeur maximum de contrainte de cisaillement $C_1$ est de préférence inférieure ou égale à 200 Pa.

Il est entendu selon ci-dessus et ci-après que les différentes valeurs de viscosité et de contrainte sont mesurées une fois le gel formé. Avant d'effectuer les mesures de viscosité et de profil rhéologique du gel, il convient de s'assurer que le gel soit bien formé et stable. Il convient donc d'attendre au moins 24 heures après la préparation du gel.

De manière préférentielle, les chutes de viscosité induites par le cisaillement sur le gel ne sont pas immédiatement réversibles, c'est à dire que le matériau ne se casse pas sous forte contrainte et reste homogène lorsqu'il a atteint sa viscosité la plus faible.

Le matériau gélifiant hydrophile selon l'invention est constitué par tout matériau gélifiant susceptible de former un gel ou une composition ayant l'apparence d'un gel et présentant le profil rhéologique selon l'invention.

Selon un mode de réalisation préférentiel de l'invention, le matériau gélifiant hydrophile est un polymère gélifiant hydrophile. Des polymères gélifiants hydrophiles utiles selon l'invention sont en particulier des polyesters sulfonés de masse moléculaire en poids inférieure à 20 000, de préférence inférieure à 15 000.

De tels polymères peuvent être plus particulièrement des oligomères copolyesters terephtaliques hydrosolubles ou hydrodispersables comprenant essentiellement des motifs répétitifs dicarboxylates de formule (I):

$$[-CO-A-CO-O-(CH_2-CH_2-O)_n] \tag{I}$$

dans laquelle

A représente un groupement 1,4-phénylène, sutfo-1,3-phénylène et éventuellement 1,3-phénylène,

n va de 1 à 4,

au moins 35 % en mole desdits motifs de formule (I) étant des motifs de formule (I) pour lesquels A représente un groupement 1,4-phénylène et n est égal à 1,

au moins 7 % en mole desdits motifs de formule (I) étant des motifs de formule (1) pour lesquels A représente un groupement sulfo-1,3-phénylène,

la masse moléculaire en poids desdits oligomères copolyesters étant inférieure à 20 000, de préférence inférieure à 15 000.

De manière préférentielle, au moins 40 % en mole, plus préférentiellement entre 40 et 90 % en mole des motifs de formule (I) sont des motifs de formule (I) pour lesquels A représente'un groupement 1,4-phénylène et n est égal à 1.

De préférence, au moins 10 % en mole, plus préférentiellement entre 10 % et 25 % en mole des motifs de formule (I) sont des motifs de formule (I) pour lesquels A représente un groupement sulfo-1,3-phénylène.

Les extrémités des chaînes desdits oligomères copolyesters peuvent être semblables ou différentes et représentées essentiellement par les groupements de formule (I'):

$$-CO-A-CO-O-(CH_2-CH_2)_n-OH \tag{I'}$$

dans laquelle A et n sont définis ci-dessus.

Lesdits oligomères peuvent également présenter en extrémités de chaîne, et ce en quantités mineures des groupements de formules

$$-A-CO-OH$$

$$-A-CO-OR$$

formules dans lesquelles A est défini ci-dessus et R représente un groupement alkyle en $C_1$-$C_4$.

Lorsque A représente un groupement sulfo-1,3-phénylène, il s'agit plus particulièrement d'un sulfonate de métaux alcalins, notamment sodium ou potassium, ou d'un sulfonate d'ammonium ou de mono-, di-, tri- ou tétra-alkylammonium inférieur. Par alkylammonium inférieur, on entend de préférence selon l'invention un ammonium dont le ou les radicaux alkyles sont des alkyles inférieurs, de préférence en $C_1$-$C_6$. De manière préférentielle, il s'agit d'un sulfonate de sodium.

L'oligomère copolyester peut comprendre éventuellement jusqu'à 20 % en mole, de préférence jusqu'à 5 % en mole de motifs de formule (I) pour lesquels A représente un groupement 1,3-phénylène.

Selon un mode de réalisation préférentiel de l'invention, l'oligomère copolyester ci-dessus a une masse moléculaire en poids comprise entre 5 000 et 14 000, plus préférentiellement comprise entre 8 000 et 10 000.

Les masses moléculaires en poids sont mesurées par chromatographie par perméation de gel dans le diméthylacétamide contenant $10^{-2}$ N de LiBr, à 100°C. Les résultats sont exprimés en équivalents polystyrène.

Lesdits oligomères copolyesters peuvent être obtenus par les procédés usuels de préparation des polyesters par voie fondue, voie solvant ou voie interfaciale, procédés faisant intervenir des réactions

.   d'estérification de diacides et de diols et polycondensation

.   de transestérification de diesters et de diols et polycondensation

.   d'autocondensation d'hydroxyacides

.   de Schotten-Baumann par mise en oeuvre de diols et de chlorures d'acide et polycondensation

.   de polymérisation de lactones

en contrôlant la teneur minimum en motifs de formule (I) pour lesquels A représente un groupement 1,4-phénylène et n est égal à 1, semblables par les rapports stoechiométriques initiaux des différents monomères et par la maîtrise des réactions secondaires.

Un mode de préparation particulièrement intéressant est celui par transestérification / polycondensation et / ou d'estérification / polycondensation par voie fondue à l'aide d'un catalyseur de transestérification et/ou estérification.

La maîtrise de la structure est obtenue par contrôle de la teneur minimum en motifs de formule (I) pour lesquels A représente un groupement 1,4-phénylène et n est égal à 1, semblables par les rapports stoechiométriques initiaux des différents monomères diacides et/ou diesters et diol et par mise en oeuvre d'un agent limiteur d'éthérification, agent limiteur qui peut être un composé basique tel que les amines aliphatiques ou aromatiques ou un hydroxyde ou acétate de métaux alcalins ou alcalino-terreux.

Le contrôle de la masse moléculaire est obtenu d'une manière connue de l'homme du métier, par compromis adéquat entre la pression, la température et le temps.

Les nouveaux oligomères copolyesters téréphtaliques faisant l'objet de l'invention peuvent être préparés par estérification et/ou transestérification / polycondensation d'une composition monomère à base:

- d'acide, anhydride ou diester téréphtalique (Tp)
- d'acide, anhydride ou diester sulfoisophtalique (Slp)
- éventuellement d'acide, anhydride ou diester isophtalique (Ip)
- et d'éthylène glycol (EG)

selon des quantités relatives correspondant à

* un rapport molaire (Slp) / [(Tp)+(Slp)+(lp)] d'au moins 7/100, de préférence d'au moins 10/100, tout particulièrement de 10/100 à 25/100
* un rapport molaire (Ip) / [(Tp)+(Slp)+(lp)] de 20/100 au plus, de préférence de 5/100 au plus
* un rapport molaire (EG) / [(Tp)+(Slp)+(lp)] de 2/1 à 3/1.

en présence d'un catalyseur d'estérification et/ou transestérification et d'un limiteur de d'éthérification.

Le monomère téréphtalique (Tp) est de préférence mis en oeuvre sous la forme de diester inférieur (diester de dialkyle en $C_1$-$C_4$), de diméthyle de préférence.

Le monomère sulfoisophtalique (Slp) est de préférence mis en oeuvre sous la forme d'un sulfonate de métal alcalin (sodium notamment) de diester inférieur (d'alkyle en $C_1$-$C_4$), de méthyle de préférence. On peut citer tout particulièrement le sodio-oxysulfonyl-5 isophtalate de diméthyle.

Le monomère isophtalique (lp) éventuel est de préférence mis en oeuvre sous la forme d'acide isophtalique.

Lorsque tous les monomères "diacides" sont mis en oeuvre sous la forme d'un diesters, l'opération de transestérification (interéchange) entre ces monomères "diacides" et l'éthylène glycol est effectuée à une température supérieure ou égale à 130°C, de préférence de l'ordre 140 à 220°C et tout particulièrement de l'ordre de 180 à 220°C ; à cette température le méthanol (cas préférentiel des diesters méthyliques) formé est éliminé du milieu réactionnel de préférence par distillation.

Cette opération d'interéchange est réalisée en présence d'un catalyseur de transestérification métallique et d'un limiteur d'éthérification. Ledit catalyseur est de préférence un carboxylate métallique, tel que l'acétate de manganèse, l'acétate de zinc, l'acétate de cobalt ou l'acétate de calcium, ou d'un titanate organique ou minéral, tel que le titanate de butyle, le titanate de nitrilo-2,2',2"-triéthyle (ou aminotriéthanolate de titane jouant en outre le rôle de limiteur d'éthérification) ou le titanate de calcium. Les catalyseurs préférés sont les titanates organiques ; ils sont mis en oeuvre en quantités de l'ordre d'au moins 0,001% en poids exprimé en titane, de préférence de l'ordre de 0,002% à 0,02% en poids de titane par rapport au poids de réactifs présents.

L'agent limiteur d'éthérification peut être un composé basique tel que les amines aliphatiques ou aromatiques (triéthanolamine, carbonate de guanidine, diméthylaniline, naphtylamine ...) ou un hydroxyde ou acétate de métaux alcalins ou alcalino-terreux (acétate de sodium, potassium, benzoate de sodium ...). Il est mis en oeuvre généralement en quantité de l'ordre de 0,001 % à 0,05% par rapport au poids de réactifs présents.

La durée de l'opération d'interéchange est de 1 à 4 heures; elle est généralement de l'ordre de 2 à 3 heures.

Lorsque plus de 90% de la quantité théorique de méthanol a été distillée, le polyol excédentaire est éliminé en portant la température du milieu réactionnel à 230°C.

L'opération de polycondensation est de préférence réalisée à une température de l'ordre de 230 à 280°C, de préférence de l'ordre de 240 à 260°C, dans un autre réacteur préalablement porté à cette température et progressivement mis sous vide jusqu'à une pression qui peut aller jusqu'à 10 Pa ; une réduction de pression jusqu'à 10 millibar environ dure de l'ordre de 40 minutes.

L'opération de polycondensation se déroule avec élimination de molécules de polyol, cette opération est stoppée lorsque le couple moteur de l'arbre d'agitation indique une valeur équivalente à environ 0,5 à 5 mètres.newton pour une température de 250°C de la masse réactionnelle et une vitesse d'agitation de 80 tours / minute d'un mobile en forme d'ancre dans un réacteur de 7,5 litres. Le vide est ensuite cassé à l'azote, et le polymère est coulé dans une lingotière ; après refroidissement, le polymère est broyé.

Lorsque l'un des monomères "diacides" est présent sous forme diacide ou anhydride et le ou les autres sous forme de diester(s), lesdits oligomères copolyesters sont obtenus en réalisant d'abord une opération de transestérification des monomères diesters avec de l'éthylène glycol dans les conditions ci-dessus décrites, suivie d'une opération d'estérification dans le milieu du monomère diacide ou anhydride avec de l'éthylène glycol, puis polycondensation dans les conditions décrites ci-dessus, la quantité totale d'éthylène glycol étant répartie entre les deux opérations (transestérification et estérification).

Si nécessaire, l'opération d'estérification est réalisée par ajout dans le milieu réactionnel résultant de l'opération de transestérification, du monomère sous forme diacide ou anhydride et d'éthylène glycol préalablement mis en suspension, à une température correspondant à celle de la fin de la température d'interéchange ; la période d'introduction est de l'ordre de 1 heure.

Cette opération d'estérification est réalisée à une température de l'ordre de 230 à 280°C, de préférence de l'ordre de 250 à 260°C, en présence d'un catalyseur du même type que celui de transestérification et d'un agent limiteur d'éthérification.

L'opération est réalisée en présence des mêmes types de catalyseur et de limiteur d'éthérification que ceux mis en oeuvre lors de l'opération de transestérifiacation, et ce dans les mêmes proportions.

La réaction s'effectue avec élimination d'eau qui est soutirée du réacteur en même temps que le polyol en excès.

Ce type de procédé de préparation est notamment décrit dans la demande de brevet WO 95/32997 (RHONE-POULENC CHIMIE).

De manière préférentielle, la composition selon l'invention comprend entre 0,5 et 15 % en poids par rapport au poids total de la composition de matériau gélifiant hydrophile, plus préférentiellement entre 2 et 10 % en poids.

La composition selon l'invention est une composition cosmétique pouvant être appliquée sur la peau, les muqueuses, les cheveux ou les phanères.

La composition selon l'invention est constituée par un gel aqueux pouvant comprendre en outre une phase grasse. La phase grasse peut comprendre des huiles, volatiles ou non, ou cires usuelles en cosmétique, d'origine animale, végétale, minérale ou synthétique, seules ou en mélanges, notamment des huiles de silicone volatiles ou non, en particulier des polysiolxanes. Dans ce cas, la phase grasse peut être dispersée dans le gel, en particulier sous la forme d'une émulsion de type huile dans eau. La quantité de phase grasse dans les compositions selon l'invention est de préférence inférieure ou égale à 10 % en poids par rapport au poids total de la composition, plus préférentiellement inférieure ou égale à 5 % en poids.

La composition selon l'invention peut comprendre des additifs et/ou des actifs usuels en cosmétique, étant entendu que l'homme du métier saura déterminer les quantités de ces additifs et actifs susceptibles d'être ajoutés à la composition selon l'invention de manière à ne pas altérer le profil rhéologique du gel la constituant.

Les additifs usuels en cosmétique sont en particulier des parfums, des colorants, des absorbeurs d'odeur, des additifs assurant la stabilité de la composition comme des conservateurs, des filtres U.V.A et/ou U.V.B., des antioxydants hydrophiles et/ou lipophiles, des chélatants, etc. Les quantités de ces différents adjuvants sont celles classiquement utilisées dans le domaine considéré, et par exemple de 0,01 à 5 % en poids du poids total de la composition. Ces adjuvants, selon leur nature, peuvent être introduits dans la phase aqueuse ou dans la phase grasse lorsque la composition comprend également une phase grasse.

La composition selon l'invention peut également comprendre des actifs usuels en cosmétique, hydrophiles et/ou lipophiles, en particulier des agents anti-radicaux libres, des alpha- ou bêta-hydroxyacides, des des filtres U.V. A et/ou U.V.B., des céramides, des agents antipelliculaires comme l'octopirox ou la pyrithione de zinc, des agents antiacnéiques comme l'acide rétinoïque ou le peroxyde de benzoyle, des agents anti-chute des cheveux comme le minoxidil, des agents antifongiques ou antiseptiques, etc. Elle peut en outre comprendre des électrolytes, plus particulièrement des sels de strontium, de magnésium ou de maganèse, comme par exemple le chlorure de strontium.

Bien entendu, la composition selon l'invention ne comprendra pas de constituants susceptibles d'altérer les propriétés rhéologiques spécifiques du gel aqueux la constituant.

La composition selon l'invention est de manière préférentielle une composition topique, cosmétique ou pharmaceutique, destinée à être appliquée sur la peau, les muqueuses, les cheveux ou les phanères.

Elle peut être employée pour toutes les utilisations dermocosmétiques usuelles, et notamment comme composition d'hygiène corporelle, comme composition capillaire, comme composition de maquillage ou comme composition de soin. De manière préférentielle, cette composition est destinée à être appliquée sur les cheveux.

Pour une utilisation comme composition capillaire, la composition selon l'invention, du fait des propriétés rhéologiques spécifiques du gel la constituant, apporte un bon effet coiffant et de la discipline dans la coiffure.

Pour obtenir un effet fixant ou améliorer l'effet coiffant et démêlant, on peut ajouter à la composition selon l'invention un matériau fixant ou un matériau conditionneur. Ces matériaux fixants ou conditionneurs peuvent être employés dans des quantités comprises entre 0,01 et 15 % en poids par rapport au poids total de la composition, de préférence entre 0,1 et 8 % en poids.

La composition selon l'invention peut également comprendre des actifs pour le soin des cheveux et/ou des agents de renfort de brillance et/ou des agents de coloration des capillaire. Ces actifs et/ou agents capillaires peuvent être employés dans des quantités comprises entre 0,01 et 20 % en poids par rapport au poids total de la composition selon l'invention.

Les matériaux fixants utiles selon l'invention sont essentiellement constitués par au moins un polymère fixant, seul ou en combinaison avec des additifs cosmétiques usuels, par exemple des plastifiants, ou des agents neutralisants. Selon l'invention, on peut utiliser tout polymère fixant connu en soi. On peut utiliser en particulier un polymère fixant choisi parmi les polymères anioniques, cationiques, amphotères, non ioniques et leurs mélanges. Les polymères fixants anioniques ou amphotères peuvent être si nécessaire neutralisés partiellement ou totalement. Les agents de neutralisation sont par exemple la soude, la potasse, l'amino-2 méthyl-2 propanol-1, la monoéthanolamine, la triéthanolamine ou la triisopropanolamine, les acides minéraux ou organiques tels que l'acide chlorhydrique ou l'acide citrique. Les polymères fixants peuvent être utilisés sous forme solubilisée ou sous forme de dispersions de particules solides de polymère.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

Les polymères fixants anioniques généralement utilisés sont des polymères comportant des groupements dérivés d'acide carboxylique, sulfonique ou phosphorique et ont un poids moléculaire moyen en poids compris entre environ 500 et 5.000.000. De tels polymères sont notamment décrits dans les brevets et demandes de brevet DE 2.330.956, FR 1.222.944, FR 1.564.110, FR 1.580.545, FR 2.198.719, FR 2.265.782, FR 2.265.781, FR 2.350.384, FR 2.357.241, FR 2.439.798, GB 839.805, LU 75370, LU 75371, US 2.047.398, US 2.723.248, US 2.102.113 et US 4.128.631. Ils sont notamment choisis parmi les produits commercialisés sous les dénominations VERSICOL® E ou K par la société ALLIED COLLOID, AMERHOLD® DR 25 par la société AMERCHOL, QUADRAMER® par la Société AMERICAN CYANAMID, ARISTOFLEX® A, LUVIFLEX® VBM 70, LUVIMER® 100 P ou MAEX ou MAE, ULTRAHOLD® et ULTRAHOLD® STRONG par la société BASF, COSMEDIA® POLYMER HSP 1180 par la société HENKEL, RETEN® 421, 423 ou 425 par la Société HERCULES; ACRYLIDONE® LM, GANTREZ® AN ou ES et AVANTAGE® CP par la société ISP, Flexan® 500, Flexan® 130 et RESINES 28-29-30, 26-13-14 ou 28-13-10 par la société NATIONAL STARCH, ACUDYNE® 255 par la société ROHM & HAAS, EUDRAGIT® L par la société ROHM PHARMA et STEPANHOLD® EXTRA par la société STEPAN, ou le copolymère acide crotonique / acétate de vinyle / t.butylbenzoate de vinyle de la société CHIMEX.

Les polymères fixants amphotères utilisables conformément à l'invention sont notamment décrits dans les brevets FR 1 400 366 et US 3 836 537. Ils sont notamment choisis parmi les produits désignés par la dénomination CTFA (4ème Ed., 1991) Octylacrylamide / acrylates / butylaminoethylmethacrylate copolymer et ceux commercialisés sous les dénominations AMPHOMER® , AMHOMER® LV 71 ou LOVOCRYL® 47 par la société NATIONAL STARCH, DIAFORMER® Z301 par la société SANDOZ et EVALSAN® par la société JAN DEKKER

Les matériaux conditionneurs utiles selon l'invention sont essentiellement constitués par les matériaux conditionneurs usuels en cosmétique. Il sont notamment choisis parmi les agents tensio-actifs cationiques, les polymères anioniques ou non ioniques ou cationiques ou amphotères, les protéines, les hydrolysats de protéines, les céramides, les pseudocéramides, les acides gras à chaînes linéaires ou ramifiées en $C_{16}$-$C_{40}$ tels que l'acide méthyl-18 eicosanoique, les silicones linéaires ou ramifiées, organomodifiées ou non, volatiles ou non volatiles, solubles et insolubles dans le milieu et leurs mélanges.

Les agents conditionneurs de type polymères cationiques utiles selon l'invention peuvent être choisis parmi tous ceux déjà connus en soi comme améliorant les propriétés cosmétiques des cheveux, à savoir notamment ceux décrits dans les demandes de brevet EP 0 337 354, FR 2 270 846, FR 2 383 660, FR 2 598 611, FR 2 470 596 et FR 2 519 863. Parmi tous les polymères cationiques susceptibles d'être utilisés dans le cadre de la présente invention, on préfère mettre en oeuvre les dérivés d'éther de cellulose quaternaires tels que les produits commercialisés sous la dénomination JR 400® par la société UNION CARBIDE CORPORATION, ou sous la dénomination CELQUAT® L 200 par la société NATIONAL STARCH, les cyclopolymères, en particulier les homopolymères de sel de diallyldiméthylammonium et les copolymères de sel de diallyldiméthylammonium et d'acrylamide en particulier les chlorures, commercialisés sous les dénominations MERQUAT® 100, MERQUAT® 550 et MERQUAT® S par la société MERCK, les polysaccharides cationiques et plus particulièrement les gommes de guar modifiées par du chlorure de 2,3-époxypropyl triméthylammonium commercialisées par exemple sous la dénomination JAGUAR C13S® par la société MEYHALL.

Parmi les agents de renfort de brillance, on peut citer les silicones arylées non volatiles, en particulier les polyalkylarylsiloxanes telles que Silicone phénylée commercialisée sous la dénomination DC 556 par la société DOW CORNING - Diphényldiméthicone commercialisée sous la dénomination MIRASIL® DPDM par la société RHONE POULENC.

Parmi les agents de coloration capillaire, on peut citer en particulier les agents de coloration directe. Parmi ceux classiquement utilisés, on peut citer des colorants nitrés benzéniques, tels que les nitrophénylènediamines, les nitrodiphénylamines, les nitroanilines, les éthers de phénol nitrés ou les nitrophénols, des nitropyridines, des colorants anthraquinoniques, mono- ou di-azoïques, triarylméthaniques, aziniques, acridiniques et xanthéniques ou encore des colorants métallifères. Ces agents de coloration directe, sous forme de base ou salifiée, sont généralement présents dans la composition selon l'invention dans des proportions pouvant aller d'environ 0,001 à environ 10%, et de préférence d'environ 0,05 à environ 5% en poids par rapport au poids total de la composition.

La composition selon l'invention est de préférence destinée à être vaporisée.

La présente invention concerne donc également un dispositif constitué par un récipient contenant une composition telle que définie ci-dessus et un moyen de distribution de ladite composition.

On pourra distinguer selon l'invention des dispositifs de type flacons pompes, pour lesquels le moyen de distribution de la composition cosmétique est une pompe et les dispositifs aérosols pour lesquels la composition cosmétique comprend en outre une quantité appropriée de propulseur, la distribution du produit étant assurée par un système approprié de valve de distribution commandée par une tête de distribution, elle même comprenant une buse par laquelle la composition aérosol est vaporisée.

Le propulseur est constitué par les gaz comprimés ou liquéfiés usuellement employés pour la préparation de compositions aérosols. On emploiera de manière préférentielle l'air, le gaz carbonique ou l'azote comprimés, ou encore un gaz soluble ou non dans la composition tel que le diméthyl éther, les hydrocarbures halogénés ou non et leurs mélanges.

La quantité de propulseur dans la composition cosmétique sera suffisante pour permettre la distribution de la composition. Elle sera de manière avantageuse comprise entre 20 et 50 % en poids par rapport au poids total de la composition.

La présente invention concerne également l'utilisation cosmétique d'une composition telle que définie ci-dessus.

Elle concerne enfin un procédé de traitement cosmétique de la peau, des muqueuses ou des phanères dans lequel on applique la composition telle que définie ci-dessus sur la peau, les muqueuses ou les phanères. De manière préférentielle, la composition est appliquée au moyen du dispositif selon l'invention.

Les exemples ci-après permettent d'illustrer l'invention, sans toutefois en limiter la portée. Les pourcentages des constituants des différentes compositions en exemples sont exprimés en poids par rapport au poids total de la composition. L'expression « ma » signifie matière active.

**Exemple 1 Préparation d'un oligomère copolyester téréphtalique**

Dans un réacteur en acier inoxydable de 7,5 litres, muni d'un agitateur à ancre tournant à 80 tr/mn relié à un couplemètre KYOWA, d'une double enveloppe pour la circulation d'un liquide caloporteur et d'une colonne à distiller régulée par une électrovanne on introduit :

- 11,47 moles de téréphtalate de diméthyle
- 2,53 moles de diméthyl-5 sulfonate de sodium
- 39,16 moles d'éthylène glycol
- 54 ppm en poids de titane, sous forme d'aminotriéthanolate de titane comme catalyseur et agent limiteur d'éthérification.

Le mélange est préchauffé à 180°C. Il est ensuite porté jusqu'à la température de 220°C en environ 130 minutes, pour distiller plus de 90% de la quantité théorique de méthanol.

Le mélange réactionnel est ensuite amené à 230°C en 30 minutes. Lorsque la masse réactionnelle a atteint cette température, on introduit en 60 minutes, toujours à 230°C, une suspension dont la composition est la suivante :

- 0,5 mole d'acide isophtalique
- 2,36 moles d'acide téréphtalique
- 8 moles d'éthylène glycol

La masse réactionnelle est alors amenée à la température de 250°C en 60 minutes.

Pendant la période d'introduction du mélange et pendant la période de chauffage jusqu'à 250°C, on distille un mélange d'eau et d'éthylène glycol sans rétrogradation.

Le mélange réactionnel est ensuite transféré dans un autoclave préchauffé à 250°C puis mis sous pression réduite de 100 millibar en 22 minutes. Après 2 minutes dans ces conditions de température et de pression, la masse réactionnelle est coulée et refroidie.

Le copolyester obtenu présente les caractéristiques structurales décrites au tableau 1 ci-après, dans lequel:

* "% molaire des motifs diacides" correspond à la teneur, en %, de chaque diacide ou diester mise en oeuvre par rapport à l'ensemble des diacides ou diesters mis en oeuvre.
    "Tp" signifie : motif téréphtalique
    "Ip" signifie : motif isophtalique
    "Slp" signifie : motif sulfoisophtalique
* Les caractéristiques de la partie "glycol" des copolyesters sont obtenues par méthanolyse des produits à 190°C pendant 16 heures suivies d'une analyse par la technique de chromatographie en phase vapeur et dosage par étalonnage interne.

    - "% molaire des motifs diols" correspond à la teneur, en %, des motifs oxyéthylène "G", des motifs di(oxyéthylène) "2G", des motifs tri(oxyéthylène) "3G" et des motifs tetra(oxyéthylène) "4G", par rapport à l'ensemble des motifs diols.

* "%GT/S motifs" correspond au % molaire des motifs de formule (1)

$$[-CO-A-CO-O-(CH_2-CH_2-O)_n-] \tag{I}$$

où A est 1,4 phénylène et n=1
par rapport à l'ensemble des motifs de formule (1)
où A est 1,4 phénylène, sulfo 1,3 phénylène et éventuellement 1,3 phénylène et n va de 1 à 4
"%GT/S motifs" se calcule par la formule suivante :

$$\%GT/S \text{ motifs} = (\% \text{ molaire de motifs Tp}) \times (\% \text{ molaire de motifs G}) / 100$$

* La masse molaire des polyesters (Mw) est déterminée par chromatographie par permation de gel (GPC) dans le DMAc/LiBr à 100%, les résultats sont données en équivalents polystyrène.

| % molaire des motifs diacides | |
|---|---|
| Tp | 82 |
| Ip | 3 |
| Slp | 15 |
| %GT/S motifs | 46,5 |
| % molaire des motifs diols | |
| G | 56,8 |
| 2G | 30,7 |
| 3G | 10 |
| 4G | 2,5 |
| Mw | 8 000 |

### Exemple 2 Profil rhéologique d'un gel à 8 % de l'oligomère de l'exemple 1

On prépare un gel aqueux en mélangeant à froid 8 % d'oligomère de l'exemple 1 et le complément à 100 % d'eau déminéralisée. Le gel fluide obtenu est coulé dans un moule et laissé reposer 24 heures. Après prise en masse, on mesure le profil rhéologique du gel obtenu:

| | |
|---|---|
| Viscosité initiale $V_0$ | 30 000 Pa.s |
| Contrainte de cisaillement $C_1$ | 80 Pa |
| Viscosité $V_2$ a une contrainte $C_2$ de 105 Pa | 30 Pa.s. |

### Exemple 3 Spray de coiffage en flacon pompe

On reprend le mode opératoire de l'exemple 2 avec les constituants suivants:

| | |
|---|---|
| Oligomère de l'exemple 1 | 5 % ma |
| Copolymère PVP/PA (65/35) | 2 % ma |
| Eau       qsp | 100 % |

Le gel obtenu est ensuite conditionné dans un dispositif constitué par un récipient et un moyen de diffusion de type pompe vendu par la société COSTER sous la référence MS P 200 Buse V06203.

La composition est diffusée sans difficulté par vaporisation pour être appliquée sur les cheveux.

### Exemple 4 Spray de conditionneur en flacon pompe

On reprend le mode opératoire de l'exemple 2 avec les constituants suivants:

| | |
|---|---|
| Oligomère de l'exemple 1 | 5 % ma |
| Polyquaternium 4 (commercialisé sous la dénomination Celquat® L200 par la société NATIONAL STARCH) | 2 % ma |
| Eau       qsp | 100 % |

Le gel obtenu est ensuite conditionné dans un dispositif constitué par un récipient et un moyen de diffusion de type pompe vendu par la société COSTER sous la référence MS P 200 Buse V06203.

La composition est diffusée sans difficulté par vaporisation pour être appliquée sur les cheveux.

### Exemple 5 Spray de conditionneur en flacon pompe

On reprend le mode opératoire de l'exemple 2 avec les constituants suivants:

| | |
|---|---|
| Oligomère de l'exemple 1 | 5 % ma |
| Polyaminosiloxane (commercialiée sous la dénomination DC 939 par la société DOW CORNING) | 2 % ma |
| Eau       qsp | 100 % |

Le gel obtenu est ensuite conditionné dans un dispositif constitué par un récipient et un moyen de diffusion de type pompe vendu par la société COSTER sous la référence MS P 200 Buse V06203.

La composition est diffusée sans difficulté par vaporisation pour être appliquée sur les cheveux.

### Exemple 6 Spray aérosol

On reprend le mode opératoire de l'exemple 2 avec les constituants suivants:

| | |
|---|---|
| Oligomère de l'exemple 1 | 5 % ma |
| Eau déminérailsée | 67 % |
| Diméthyl éther       qsp | 100 % |

Le gel obtenu est ensuite conditionné dans un dispositif constitué par un récipient et un moyen de diffusion de type aérosol, lui même constitué par une valve référence C21326002 de la société PRECISION et un bouton poussoir référence 31696440AD87 de la société PRECISION.

La composition est diffusée sans difficulté par vaporisation pour être appliquée sur les cheveux.

### Revendications

**1.** Composition topique constituée par un gel aqueux comprenant un matériau gélifiant hydrophile, le dit gel présentant le profil rhéologique suivant:

- une viscosité initiale $V_0$ comprise entre 3000 et 50000 Pa.s, la dite viscosité initiale $V_0$ étant stable jusqu'à une contrainte de cisaillement $C_1$
- une viscosité $V_2$ après cisaillement à une contrainte $C_2$ pour laquelle le rapport $V_0/V_2$ est supérieur ou égal à 1000,
- la différence $C_2-C_1$ étant inférieure ou égale à 100 Pa.

2. Composition selon la revendication 1, **caractérisée en ce que** le gel présente une viscosité V, mesurée à la containte de cisaillement $C_1$, le rapport $V_0/V_1$ étant inférieur ou égal à 2.

3. Composition selon l'une des revendications 1 ou 2, **caractérisée en ce que** la contrainte de cisaillement $C_1$ est supérieure ou égale à 50 Pa.

4. Composition selon l'une des revendications 1 à 3, **caractérisée en ce que** les chutes de viscosité induites par le cisaillement sur le gel ne sont pas immédiatement réversibles.

5. Composition selon l'une des revendications 1 à 4, **caractérisée en ce que** le matériau gélifiant hydrophile est un polymère gélifiant hydrophile.

6. Composition selon la revendication 5, **caractérisée en ce que** le polymère gélifiant hydrophile est un oligomère copolyester terephtalique hydrosoluble ou hydrodispersable comprenant essentiellement des motifs répétitifs dicarboxylates de formule (I):

$$[-CO-A-CO-O-(CH_2-CH_2-O)_n] \qquad\qquad (I)$$

dans laquelle

A représente un groupement 1,4-phénylène, sulfo-1,3-phénylène et éventuellement 1,3-phénylène,

n va de 1 à 4,

au moins 35 % en mole, préférentiellement au moins 40 % en mole, plus préférentiellement entre 40 et 90 % en mole desdits motifs de formule (I) étant des motifs de formule (I) pour lesquels A représente un groupement 1,4-phénylène et n est égal à 1,

au moins 7 % en mole, préférentiellement au moins 10 % en mole, plus préférentiellement entre 10 % et 25 % en mole desdits motifs de formule (I) étant des motifs de formule (I) pour lesquels A représente un groupement sulfo-1,3-phénylène,

l'oligomère copolyester pouvant comprendre éventuellement jusqu'à 20 % en mole, de préférence jusqu'à 5 % en mole de motifs de formule (I) pour lesquels A représente un groupement 1,3-phénylène, et

la masse moléculaire en poids dudit oligomère copolyester étant inférieure à 20 000, de préférence inférieure à 15 000.

7. Composition selon la revendication 6, **caractérisée en ce que** l'oligomère copolyester a une masse moléculaire en poids comprise entre 5 000 et 14 000, plus préférentiellement comprise entre 8 000 et 10 000.

8. Composition selon l'une des revendications 1 à 7, **caractérisée en ce qu'**elle comprend entre 0,5 et 15 % en poids par rapport au poids total de la composition de matériau gélifiant hydrophile, plus préférentiellement entre 2 et 10 % en poids.

9. Composition selon l'une des revendications 1 à 8, **caractérisée en ce qu'**il s'agit d'une composition capillaire.

10. Composition selon la revendication 9, **caractérisée en ce qu'**elle comprend un matériau fixant et/ou un matériau conditionneur et/ou des actifs pour le soin des cheveux et/ou des agents de renfort de brillance et/ou des agents de coloration des capillaire.

11. Dispositif constitué par un récipient contenant une composition selon l'une des revendications 1 à 10 et un moyen de distribution de ladite composition.

12. Dispositif selon la revendication 11, **caractérisé en ce que** le moyen de distribution de la composition cosmétique est une pompe.

**13.** Dispositif selon la revendication 11, **caractérisé en ce que** la composition cosmétique comprend en outre une quantité appropriée de propulseur, la distribution du produit étant assurée par un système approprié de valve de distribution commandée par une tête de distribution, elle même comprenant une buse par laquelle la composition aérosol est vaporisée.

**14.** Dispositif selon la revendication 13, **caractérisé en ce que** le propulseur est constitué par un gaz comprimé ou liquéfié, en particulier choisi parmi l'air, le gaz carbonique ou l'azote comprimés, ou encore un gaz soluble ou non dans la composition tel que le diméthyl éther, les hydrocarbures halogénés ou non et leurs mélanges.

**15.** Dispositif selon l'une des revendications 13 ou 14, **caractérisé en ce que** la quantité de propulseur est comprise entre 20 et 50 % en poids par rapport au poids total de la composition.

**16.** Procédé de traitement cosmétique de la peau, des muqueuses ou des phanères dans lequel on applique la composition selon l'une des revendications 1 à 10 sur la peau, les muqueuses ou les phanères.

**17.** Procédé selon la revendication 16, **caractérisé en ce que** l'on applique la composition au moyen d'un dispositif selon l'une des revendications 11 à 15.


**Patentansprüche**

**1.** Zusammensetzung zur topischen Anwendung, die aus einem wässrigen Gel besteht, das ein hydrophiles gelbildendes Material enthält, wobei das Gel das folgende rheologische Profil aufweist:

- eine anfängliche Viskosität $V_0$ im Bereich von 3 000 bis 50 000 Pa·s, wobei die anfängliche Viskosität $V_0$ bis zu einer Scherspannung Ci stabil ist,
- eine Viskosität $V_2$ nach Scherung mit einer Scherspannung $C_2$, wobei das Verhältnis $V_0/V_2$ mindestens 1 000 beträgt,
- wobei die Differenz $C_2-C_1$ höchstens 100 Pa beträgt.

**2.** Zusammensetzung nach Anspruch 1, **dadurch gekennzeichnet, dass** das Gel eine bei der Scherspannung $C_1$ gemessene Viskosität $V_1$ aufweist, wobei das Verhältnis $V_0/V_1$ höchstens 2 ist.

**3.** Zusammensetzung nach einem der Ansprüche 1 oder 2, **dadurch gekennzeichnet, dass** die Scherspannung $C_1$ mindestens 50 Pa beträgt.

**4.** Zusammensetzung nach einem der Ansprüche 1 bis 3, **dadurch gekennzeichnet, dass** das Sinken der Viskosität, das durch die Scherung des Gels verursacht wird, nicht unmittelbar umkehrbar ist.

**5.** Zusammensetzung nach einem der Ansprüche 1 bis 4, **dadurch gekennzeichnet, dass** das hydrophile gelbildende Material ein hydrophiles gelbildendes Polymer ist.

**6.** Zusammensetzung nach Anspruch 5, **dadurch gekennzeichnet, dass** das hydrophile gelbildende Polymer ein in Wasser löslicher oder in Wasser dispergierbarer, oligomerer Terephthalcopolyester ist, der im Wesentlichen wiederkehrende Dicarboxylateinheiten der Formel (I) enthält:

$$[-CO-A-CO-O-(CH_2-CH_2-O)n] \qquad (I),$$

worin bedeuten:

A 1,4-Phenylen, Sulfo-1,3-phenylen und gegebenenfalls 1,3-Phenylen, und
n 1 bis 4,

wobei mindestens 35 Mol-%, vorzugsweise mindestens 40 Mol-% und noch bevorzugter 40 bis 90 Mol-% der Einheiten der Formel (I) Einheiten der Formel (I) sind, worin A eine 1,4-Phenylengruppe bedeutet und n 1 ist, wobei mindestens 7 Mol-%, vorzugsweise mindestens 10 Mol-% und noch bevorzugter 10 bis 25 Mol-% der Einheiten der Formel (I) Einheiten der Formel (I) sind, worin A eine Sulfo-1,3-phenylengruppe bedeutet,
wobei der oligomere Copolyester gegebenenfalls bis zu 20 Mol-%, vorzugsweise bis zu 5 Mol-% Einheiten der Formel (I) enthalten kann, worin A eine 1,3-Phenylengruppe bedeutet, und
wobei das Molekulargewicht des oligomeren Copolyesters unter 20 000 und vorzugsweise unter 15 000 liegt.

**7.** Zusammensetzung nach Anspruch 6, **dadurch gekennzeichnet, dass** der oligomere Copolyester ein Molekulargewicht von 5 000 bis 14 000 und vorzugsweise 8 000 bis 10 000 aufweist.

**8.** Zusammensetzung nach einem der Ansprüche 1 bis 7, **dadurch gekennzeichnet, dass** sie 0,5 bis 15 Gew.-% hydrophiles gelbildendes Material und vorzugsweise 2 bis 10 Gew.-% hydrophiles gelbildendes Material, bezogen auf das Gesamtgewicht der Zusammensetzung, enthält.

**9.** Zusammensetzung nach einem der Ansprüche 1 bis 8, **dadurch gekennzeichnet, dass** es sich um eine Zusammensetzung für die Haarbehandlung handelt.

**10.** Zusammensetzung nach Anspruch 9, **dadurch gekennzeichnet, dass** sie einen fixierenden Stoff und/ oder ein Konditioniermittel und/oder Wirkstoffe für die Pflege der Haare und/oder Mittel zur Verbesserung des Glanzes und/ oder Haarfärbemittel enthält.

**11.** Vorrichtung, die aus einem Behälter, der eine Zusammensetzung nach einem der Ansprüche 1 bis 10 enthält, und einer Einrichtung zur Verteilung der Zusammensetzung besteht.

**12.** Vorrichtung nach Anspruch 11, **dadurch gekennzeichnet, dass** die Einrichtung zur Verteilung der kosmetischen Zusammensetzung eine Pumpe ist.

**13.** Vorrichtung nach Anspruch 11, **dadurch gekennzeichnet, dass** die kosmetische Zusammensetzung außerdem eine geeignete Menge Treibmittel enthält, wobei die Verteilung des Produktes durch ein geeignetes Ventilsystem gewährleistet wird, das über einen Verteilerkopf betätigt wird, welcher eine Düse umfasst, durch die die Aerosolzusammensetzung zerstäubt wird.

**14.** Vorrichtung nach Anspruch 13, **dadurch gekennzeichnet, dass** das Treibmittel aus einem komprimierten oder verflüssigten Gas, das insbesondere unter komprimierter Luft, komprimiertem Kohlendioxid oder komprimiertem Stickstoff ausgewählt ist, oder einem in der Zusammensetzung löslichen oder unlöslichen Gas, wie Dimethylether, gegebenenfalls halogenierten Kohlenwasserstoffen, und deren Gemischen besteht.

**15.** Vorrichtung nach einem der Ansprüche 13 oder 14, **dadurch gekennzeichnet, dass** der Mengenanteil des Treibmittels im Bereich von 20 bis 50 Gew.-%, bezogen auf das Gesamtgewicht der Zusammensetzung, liegt.

**16.** Verfahren zur kosmetischen Behandlung der Haut, der Schleimhäute oder der Hautanhangsgebilde, wobei die Zusammensetzung nach einem der Ansprüche 1 bis 10 auf die Haut, die Schleimhäute oder die Hautanhangsgebilde aufgebracht wird.

**17.** Verfahren nach Anspruch 16, **dadurch gekennzeichnet, dass** die Zusammensetzung unter Verwendung einer Vorrichtung nach einem der Ansprüche 11 bis 15 aufgetragen wird.

**Claims**

**1.** Topical composition consisting of an aqueous gel comprising a hydrophilic gelling material, the said gel having the following rheological profile:

- an initial viscosity $V_0$ of between 3000 and 50,000 Pa.s, the said initial viscosity $V_0$ being stable up to a shear strain $C_1$,
- a viscosity $V_2$ after shear at a strain $C_2$ for which the ratio $V_0/V_2$ is greater than or equal to 1000,
- the difference $C_2-C_1$. being less than or equal to 100 Pa.

**2.** Composition according to Claim 1, **characterized in that** the gel has a viscosity $V_1$ measured at the shear strain $C_1$, the ratio $V_0/V_1$ being less than or equal to 2.

3. Composition according to either of Claims 1 and 2, **characterized in that** the shear strain $C_1$ is greater than or equal to 50 Pa.

4. Composition according to one of Claims 1 to 3, **characterized in that** the falls in viscosity induced by the shear on the gel are not immediately reversible.

5. Composition according to one of Claims 1 to 4, **characterized in that** the hydrophilic gelling material is a hydrophilic gelling polymer.

6. Composition according to Claim 5, **characterized in that** the hydrophilic gelling polymer is a water-soluble or water-dispersible terephthalic copolyester oligomer essentially comprising dicarboxylate repeating units of formula (I):

$$[-CO-A-CO-O-(CH_2-CH_2-O)_n] \tag{I}$$

in which
    A represents a 1,4-phenylene, sulpho-1,3-phenylene and optionally 1,3-phenylene group,
    n ranges from 1 to 4,
    at least 35 mol%, preferably at least 40 mol%, more preferably between 40 and 90 mol% of the said units of formula (I) being units of formula (I) for which A represents a 1,4-phenylene group and n is equal to 1,
    at least 7 mol%, preferably at least 10 mol%, more preferably between 10 and 25 mol% of the said units of formula (I) being units of formula (I) for which A represents a sulpho-1,3-phenylene group,
    it being possible for the copolyester oligomer to optionally comprise up to 20 mol%, preferably up to 5 mol%, of units of formula (I) for which A represents a 1,3-phenylene group, and
    the weight-average molecular mass of the said copolyester oligomer being less than 20,000, preferably less than 15,000.

7. Composition according to Claim 6, **characterized in that** the copolyester oligomer has a weight-average molecular mass of between 5000 and 14,000, more preferably of between 8000 and 10,000.

8. Composition according to one of Claims 1 to 7, **characterized in that** it comprises between 0.5 and 15% by weight, relative to the total weight of the composition, of hydrophilic gelling material, more preferably between 2 and 10% by weight.

9. Composition according to one of Claims 1 to 8, **characterized in that** it is a hair composition.

10. Composition according to Claim 9, **characterized in that** it comprises a fixing material and/or a conditioning material and/or active agents for haircare and/or sheen-enhancing agents and/or hair dyes.

11. Device consisting of a container containing a composition according to one of Claims 1 to 10 and a means for distributing the said composition.

12. Device according to Claim 11, **characterized in that** the means for distributing the cosmetic composition is a pump-dispenser.

13. Device according to Claim 11, **characterized in that** the cosmetic composition also comprises a suitable amount of propellant, the product being distributed by means of an appropriate distribution valve system controlled by a distribution head, itself comprising a nozzle via which the aerosol composition is vaporized.

14. Device according to Claim 13, **characterized in that** the propellant consists of a compressed or liquefied gas chosen in particular from compressed air, carbon dioxide or nitrogen, or alternatively a gas which is soluble or insoluble in the composition, such as dimethyl ether, halogenated or non-halogenated hydrocarbons, and mixtures thereof.

15. Device according to either of Claims 13 and 14, **characterized in that** the amount of propellant is between 20 and 50% by weight relative to the total weight of the composition.

16. Cosmetic treatment process for the skin, mucous membranes or superficial body growths, in which the composition according to one of Claims 1 to 10 is applied to the skin, mucous membranes or superficial body growths.

17. Process according to Claim 16, **characterized in that** the composition is applied by means of a device according to one of Claims 11 to 15.